# EUROPEAN PATENT APPLICATION

(11) **EP 3 747 620 A1**
(43) Date of publication of application: **09.12.2020**
(21) Application number: 19748371.2
(22) Date of filing: 23.01.2019
(51) Int. Cl.: B29C 48/345, A61F 13/15, A61F 13/51, B29C 48/88, B29C 48/92, B32B 5/24

(54) **METHOD AND APPARATUS FOR MANUFACTURING ELASTIC SHEET, METHOD AND APPARATUS FOR MANUFACTURING STRETCHABLE COMPOSITE SHEET, AND STRETCHABLE COMPOSITE SHEET**

(30) Priority: 31.01.2018 JP 2018014325; 10.04.2018 JP 2018075760
(71) Applicant: Zuiko Corporation, Osaka 566-0045 (JP)
(72) Inventor: KOSHIJIMA Miwa, Settu-Shi, Osaka 566-0045 (JP); NAKAMURA Hideyuki, Settu-Shi, Osaka 566-0045 (JP)
(74) Representative: Dr. Gassner & Partner mbB
(86) International application number: PCT/JP2019/001985
(87) International publication number: WO 2019/151064

(57) **Abstract**

Provided are: a method and apparatus for manufacturing a highly permeable elastic sheet; a method and apparatus for manufacturing a highly gas-permeable stretchable composite sheet; and a highly gas-permeable stretchable composite sheet.

An elastic resin material containing a thermoplastic elastic resin as a main component is melted by being heated to a temperature higher than a temperature range in which the elastic resin material is elastically deformed, the molten elastic resin material is discharged in a fibrous or linear form, the discharged elastic resin material (intermediate (2)) is applied in a net shape onto a cooling member (30), the applied elastic resin material is cooled by the cooling member (30) to the temperature region in which the elastic resin material is elastically deformed, and the applied elastic resin material is solidified to obtain an elastic sheet (3). The elastic sheet (3) and non-woven fabrics (4, 6) are stacked and joined to obtain a stretchable composite sheet (8).

## Description

### Technical Field

The present invention relates to elastic sheet manufacturing methods and apparatuses, stretchable composite sheet manufacturing methods and apparatuses, and stretchable composite sheets. The present invention more particularly relates to an elastic sheet manufacturing method and apparatus, a stretchable composite sheet manufacturing method and apparatus, and a stretchable composite sheet that are suitable for use for articles, such as disposable wearable articles.

### Background Art

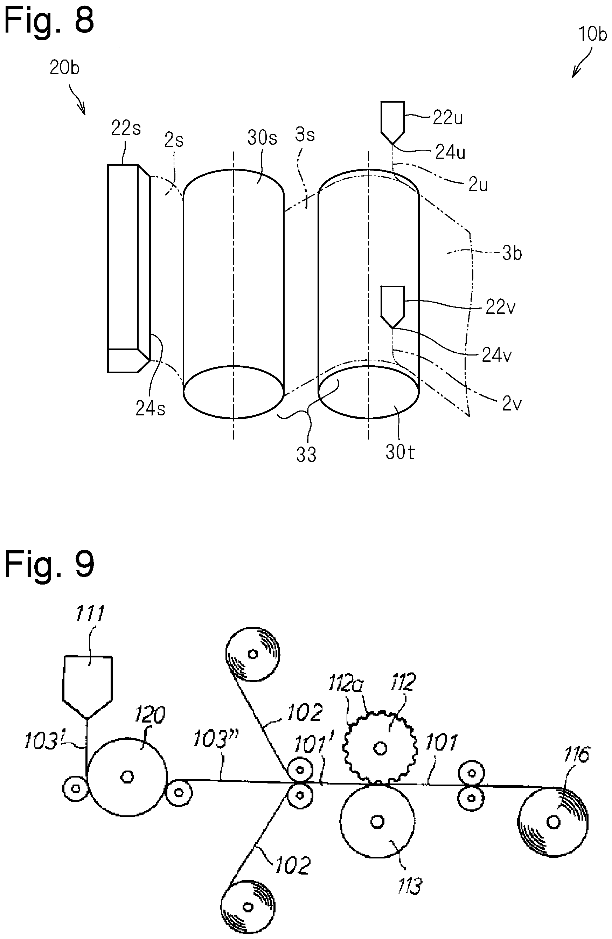

Stretchable composite sheets having high stretchability are used for, for example, back sheets of disposable diapers. FIG. 9 is a schematic diagram illustrating a stretchable composite sheet manufacturing method. As illustrated in FIG. 9, a thermoplastic elastic resin is extruded from a T-die 111 so as to form a filmy object 103' in a molten state. The filmy object 103' is subsequently cooled by a chill roller 120. A cooled filmy object 103" is sandwiched between two continuous non-woven fabrics 102 so as to provide a laminated body 101', and then the laminated body 101' is subjected to an embossing process. The embossing process involves pressurizing and sandwiching the laminated body 101' between an embossing roller 112 whose surface is dotted with a large number of projections 112a and a back-up roller 113. Performing the embossing process provides a stretchable composite sheet 101 whose layers are bonded to each other and are thus integral with each other. The stretchable composite sheet 101 is then rolled up by a roll-up roller 116. Fabrics stretchable at least in a lateral direction (which is perpendicular to a direction in which the non-woven fabrics 102 flow during manufacturing process and perpendicular to the thickness direction of the non-woven fabrics 102) are used as the non-woven fabrics 102 (see, for example, Patent Literature 1).

### Citation List

### Patent Literature

[Patent Literature 1] Japanese Patent No. 3054930

### Summary of the Invention

### Problem to be Solved by the Invention

There may be a desire to use a stretchable composite sheet having high breathability for, for example, a disposable wearable article.

The stretchable composite sheet 101 manufactured by the method illustrated in FIG. 9, however, includes the filmy object 103", making it difficult to make the stretchable composite sheet 101 breathable.

In view of these circumstances, a problem to be solved by the present invention is to provide an elastic sheet manufacturing method and apparatus capable of manufacturing a highly breathable elastic sheet, a stretchable composite sheet manufacturing method and apparatus capable of manufacturing a stretchable composite sheet having high breathability, and a stretchable composite sheet having high breathability.

### Means for Solving the Problem

To solve the above-mentioned problem, the present invention provides an elastic sheet manufacturing method as follows:
An elastic sheet manufacturing method includes: (i) a first step involving heating a first elastic resin material to a temperature higher than a first temperature range in which the first elastic resin material elastically deforms, such that the first elastic resin material is molten, the first elastic resin material being composed mainly of a first thermoplastic elastic resin; (ii) a second step involving discharging the molten first elastic resin material in a fibrous or linear form from a first discharge port; and (iii) a third step involving applying the first elastic resin material, which has been discharged from the first discharge port, in a net form to a cooling member, and cooling the applied first elastic resin material to the first temperature range by the cooling member such that the first elastic resin material is solidified.

The above method is able to provide a net-shaped elastic sheet including the first elastic resin material cooled and solidified and thus enables manufacture of an elastic sheet having high breathability. The above method also enables manufacture of an elastic sheet stretchable in all directions.

Preferably, the second step involves discharging the first elastic resin material in a linear form. The third step involves applying the first elastic resin material, which has been discharged in the linear form, in a serpentine pattern or a spiral pattern to the cooling member.

In this case, the first elastic resin material is applied in a net form with ease.

Preferably, the second step involves discharging the first elastic resin material from each of a plurality of the first discharge ports. The third step involves applying the first elastic resin materials, which have been discharged from the first discharge ports, to the cooling member such that the first elastic resin materials partially overlap with each other.

In this case, a wide net-shaped elastic sheet is easily manufacturable.

Preferably, the second step involves discharging the first elastic resin materials from the first discharge ports such that each amount of the first elastic resin materials discharged from one or plural of the first discharge ports differs from each amount of the first elastic resin materials discharged from the other of the first discharge ports.

In this case, the method enables manufacture of an elastic sheet having different stretching characteristics at different positions in a width direction (which is perpendicular to the flow direction of the elastic sheet during manufacturing process and perpendicular to the thickness direction of the elastic sheet).

Preferably, the second step involves periodically changing an amount or amounts of the first elastic resin material or materials to be discharged.

In this case, the method enables manufacture of an elastic sheet having different stretching characteristics at different positions in the flow direction (which is perpendicular to the width direction and the thickness direction).

Preferably, the first step includes a first sub-step involving heating second elastic resin materials, each composed mainly of a second thermoplastic elastic resin, to a temperature higher than a second temperature range in which the second elastic resin materials elastically deform, such that the second elastic resin materials are molten. The second step includes a second sub-step involving discharging the molten second elastic resin materials in a linear form from a plurality of second discharge ports. The third step includes a third sub-step involving: applying the second elastic resin materials, which have been discharged from the second discharge ports, in a linear form to the cooling member such that the second elastic resin materials are in parallel with each other and overlap with the first elastic resin material or materials applied to the cooling member; and cooling the applied second elastic resin materials to the second temperature range by the cooling member such that the second elastic resin materials are solidified.

In this case, the method is able to provide a net-shaped elastic sheet including the first and second elastic resin materials cooled and solidified. Changing the amount or amounts of first elastic resin material or materials (e.g., the thickness of each first elastic resin material and/or the number of first elastic resin materials) or an application pattern thereof makes it possible to adjust stretching stress mainly in the width direction of the elastic sheet. Changing the amounts of second elastic resin materials (e.g., the thickness of each second elastic resin material and/or the number of second elastic resin materials) makes it possible to adjust stretching stress mainly in the flow direction of the elastic sheet during manufacturing process (which is perpendicular to the width direction and the thickness direction of the elastic sheet). Consequently, the method facilitates adjustment of stretching stress in the flow direction and the width direction of the elastic sheet.

The first and second thermoplastic elastic resins may be identical or different from each other. When the first and second thermoplastic elastic resins are identical, the first and second elastic resin materials may be identical or different from each other.

Preferably, the first step includes a first sub-step involving heating a second elastic resin material, which is composed mainly of a second thermoplastic elastic resin, to a temperature higher than a second temperature range in which the second elastic resin material elastically deforms, such that the second elastic resin material is molten. The second step includes a second sub-step involving discharging the molten second elastic resin material in a linear or fibrous form from a second discharge port. The third step includes a third sub-step involving: delivering the first elastic resin material or materials, which has or have been cooled to the first temperature range such that the first elastic resin material or materials is or are solidified, out of the cooling member; causing the first elastic resin material or materials to pass along an additional cooling member; applying the second elastic resin material, which has been discharged from the second discharge port, in a linear or net form to the additional cooling member such that the second elastic resin material overlaps with a portion or portions of the first elastic resin material or materials extending along the additional cooling member; and cooling the applied second elastic resin material to the second temperature range by the additional cooling member such that the second elastic resin material is solidified.

In this case, the method facilitates adjustment of stretching stress in the flow direction and the width direction of the elastic sheet. Because the first and second elastic resin materials are separately cooled and solidified, the method is more likely to make a difference in stretching rate than when the first and second elastic resin materials are simultaneously cooled and solidified.

The first and second thermoplastic elastic resins may be identical or different from each other. When the first and second thermoplastic elastic resins are identical, the first and second elastic resin materials may be identical or different from each other.

The present invention provides a stretchable composite sheet manufacturing method having features described below.

The stretchable composite sheet manufacturing method includes laminating and bonding an elastic sheet to a first nonwoven fabric, the elastic sheet being manufactured by any one of the elastic sheet manufacturing methods described above.

The above method involves bonding the elastic sheet having high breathability to the first nonwoven fabric and thus enables manufacture of a stretchable composite sheet having high breathability.

When the first and second elastic resin materials are used, the second elastic resin material(s) discharged from the second discharge port(s) may be applied in a linear or net form to the additional cooling member such that the second elastic resin material(s) does or do not overlap with portion(s) of the first elastic resin material(s) extending along the additional cooling member.

Preferably, the method includes: laminating a second nonwoven fabric to the elastic sheet and the first nonwoven fabric such that the elastic sheet is sandwiched between the first nonwoven fabric and the second nonwoven fabric; and bonding the second nonwoven fabric to at least either one of the elastic sheet and the first nonwoven fabric.

In this case, the method enables manufacture of a stretchable composite sheet in which the elastic sheet is sandwiched between the first nonwoven fabric and the second nonwoven fabric.

Preferably, the method includes ultrasonically bonding the elastic sheet to the first nonwoven fabric at a plurality of locations away from each other.

When the elastic resin material(s) for the elastic sheet has or have adhesion properties similar to those of a hot-melt adhesive, the elastic sheet is easily bondable to the first nonwoven fabric. This facilitates both of bonding between the elastic sheet and the first nonwoven fabric and bonding between the first nonwoven fabric and the second nonwoven fabric.

Preferably, the method includes stretching the elastic sheet, and laminating and bonding the elastic sheet in a stretched state to the first nonwoven fabric.

In this case, bringing the elastic sheet out of the stretched state provides a stretchable composite sheet in which the surface of the first nonwoven fabric is uneven.

Preferably, the method includes laminating and bonding the elastic sheet to the first nonwoven fabric, and then stretching the elastic sheet together with the first nonwoven fabric.

In this case, the method is able to provide a stretchable composite sheet in which the surface of the first nonwoven fabric is kept substantially even after the elastic sheet is brought out of the stretched state.

Preferably, the method includes laminating and bonding the elastic sheet to the first nonwoven fabric stretchable in at least one direction, such that the one direction in which the first nonwoven fabric is stretchable is perpendicular to a direction in which the elastic sheet is to be stretched.

In this case, the method enables manufacture of a stretchable composite sheet stretchable in all directions.

To achieve the above object, the present invention provides an elastic sheet manufacturing apparatus having features described below.

The elastic sheet manufacturing apparatus includes: (a) an applicator including a first discharger; and (b) a cooling member disposed below the first discharger. The first discharger includes a first discharge port to discharge a first elastic resin material in a fibrous or linear form, the first elastic resin material being composed mainly of a first thermoplastic elastic resin, the first elastic resin material being heated to a temperature higher than a first temperature range in which the first elastic resin material elastically deforms, such that the first elastic resin material is molten. The applicator applies the first elastic resin material, which has been discharged from the first discharge port, in a net form to the cooling member. The cooling member cools the first elastic resin material, which has been applied to the cooling member, to the first temperature range such that the first elastic resin material is solidified.

The apparatus having the above features is able to provide a net-shaped elastic sheet and is thus able to manufacture a highly breathable elastic sheet. The apparatus is able to manufacture an elastic sheet stretchable in all directions.

Preferably, the applicator includes a second discharger. The cooling member is disposed below the second discharger. The second discharger includes a plurality of second discharge ports to discharge second elastic resin materials in a linear form. The second elastic resin materials are each composed mainly of a second thermoplastic elastic resin. The second elastic resin materials are heated to a temperature higher than a second temperature range in which the second elastic resin materials elastically deform, such that the second elastic resin materials are molten. The applicator applies the second elastic resin materials, which have been discharged from the second discharge ports, in a linear form to the cooling member such that the second elastic resin materials are in parallel with each other and overlap with the first elastic resin material applied to the cooling member. The cooling member cools the second elastic resin materials, which have been applied to the cooling member, to the second temperature range such that the second elastic resin materials are solidified.

In this case, the apparatus is able to provide a net-shaped elastic sheet including the first and second elastic resin materials cooled and solidified. Changing the amount of first elastic resin material (e.g., the thickness of each first elastic resin material and/or the number of first elastic resin materials) or an application pattern thereof makes it possible to adjust stretching stress mainly in the width direction of the elastic sheet. Changing the amounts of second elastic resin materials (e.g., the thickness of each second elastic resin material and/or the number of second elastic resin materials) makes it possible to adjust stretching stress mainly in the flow direction of the elastic sheet during manufacturing process. Consequently, the apparatus facilitates adjustment of stretching stress in the flow direction and the width direction of the elastic sheet.

The first and second thermoplastic elastic resins may be identical or different from each other. When the first and second thermoplastic elastic resins are identical, the first and second elastic resin materials may be identical or different from each other.

Preferably, the elastic sheet manufacturing apparatus further includes an additional cooling member. The first elastic resin material, which has been applied to the cooling member and cooled to the first temperature range such that the first elastic resin material is solidified, is delivered out of the cooling member and then passed along the additional cooling member. The applicator includes a second discharger. The additional cooling member is disposed below the second discharger. The second discharger includes a second discharge port to discharge a second elastic resin material in a linear or fibrous form. The second elastic resin material is composed mainly of a second thermoplastic elastic resin. The second elastic resin material is heated to a temperature higher than a second temperature range in which the second elastic resin material elastically deforms, such that the second elastic resin material is molten. The applicator applies the second elastic resin material, which has been discharged from the second discharge port, in a linear or net form to the additional cooling member such that the second elastic resin material overlaps with a portion of the first elastic resin material extending along the additional cooling member. The additional cooling member cools the second elastic resin material, which has been applied to the additional cooling member, to the second temperature range such that the second elastic resin material is solidified.

In this case, the apparatus facilitates adjustment of stretching stress in the flow direction and the width direction of the elastic sheet. Because the first and second elastic resin materials are separately cooled and solidified, the apparatus is more likely to make a difference in stretching rate than when the first and second elastic resin materials are simultaneously cooled and solidified.

The first and second thermoplastic elastic resins may be identical or different from each other. When the first and second thermoplastic elastic resins are identical, the first and second elastic resin materials may be identical or different from each other.

To achieve the above object, the present invention provides a stretchable composite sheet manufacturing apparatus having features described below.

The stretchable composite sheet manufacturing apparatus includes: (a) an applicator including a first discharger; (b) a cooling member disposed below the first discharger; and (c) a laminator. The first discharger includes a first discharge port to discharge a first elastic resin material in a fibrous or linear form. The first elastic resin material is composed mainly of a first thermoplastic elastic resin. The first elastic resin material is heated to a temperature higher than a first temperature range in which the first elastic resin material elastically deforms, such that the first elastic resin material is molten. The applicator applies the first elastic resin material, which has been discharged from the first discharge port, in a net form to the cooling member. The cooling member cools the first elastic resin material, which has been applied to the cooling member, to the first temperature range such that the first elastic resin material is solidified so as to form a net-shaped elastic sheet. The laminator laminates and bonds the elastic sheet to a nonwoven fabric.

The apparatus having the above features bonds the elastic sheet having high breathability to the nonwoven fabric and is thus able to manufacture a stretchable composite sheet having high breathability.

Preferably, the applicator includes a second discharger. The cooling member is disposed below the second discharger. The second discharger includes a plurality of second discharge ports to discharge second elastic resin materials in a linear form. The second elastic resin materials are each composed mainly of a second thermoplastic elastic resin. The second elastic resin materials are heated to a temperature higher than a second temperature range in which the second elastic resin materials elastically deform, such that the second elastic resin materials are molten. The applicator applies the second elastic resin materials, which have been discharged from the second discharge ports, in a linear form to the cooling member such that the second elastic resin materials are in parallel with each other and overlap with the first elastic resin material applied to the cooling member. The cooling member cools the second elastic resin materials, which have been applied to the cooling member, to the second temperature range such that the second elastic resin materials are solidified.

In this case, the apparatus is able to provide the net-shaped elastic sheet including the first and second elastic resin materials cooled and solidified. Changing the amount of first elastic resin material (e.g., the thickness of each first elastic resin material and/or the number of first elastic resin materials) or an application pattern thereof makes it possible to adjust stretching stress mainly in the width direction of the elastic sheet. Changing the amounts of second elastic resin materials (e.g., the thickness of each second elastic resin material and/or the number of second elastic resin materials) makes it possible to adjust stretching stress mainly in the flow direction of the elastic sheet during manufacturing process. Consequently, the apparatus facilitates adjustment of stretching stress in the flow direction and the width direction of the elastic sheet.

The first and second thermoplastic elastic resins may be identical or different from each other. When the first and second thermoplastic elastic resins are identical, the first and second elastic resin materials may be identical or different from each other.

Preferably, the stretchable composite sheet manufacturing apparatus further includes an additional cooling member along which the elastic sheet delivered out of the cooling member passes. The applicator includes a second discharger. The additional cooling member is disposed below the second discharger. The second discharger includes a second discharge port to discharge a second elastic resin material in a linear or fibrous form. The second elastic resin material is composed mainly of a second thermoplastic elastic resin. The second elastic resin material is heated to a temperature higher than a second temperature range in which the second elastic resin material elastically deforms, such that the second elastic resin material is molten. The applicator applies the second elastic resin material, which has been discharged from the second discharge port, in a linear or net form to the additional cooling member. The additional cooling member cools the second elastic resin material, which has been applied to the additional cooling member, to the second temperature range such that the second elastic resin material is solidified.

In this case, the apparatus facilitates adjustment of stretching stress in the flow direction and the width direction of the elastic sheet. Because the first and second elastic resin materials are separately cooled and solidified, the apparatus is more likely to make a difference in stretching rate than when the first and second elastic resin materials are simultaneously cooled and solidified.

The first and second thermoplastic elastic resins may be identical or different from each other. When the first and second thermoplastic elastic resins are identical, the first and second elastic resin materials may be identical or different from each other.

Preferably, the stretchable composite sheet manufacturing apparatus further includes a stretcher to stretch the elastic sheet. The laminator bonds the stretched elastic sheet to the nonwoven fabric.

In this case, bringing the elastic sheet out of the stretched state provides a stretchable composite sheet in which the surface of the nonwoven fabric is uneven.

To achieve the above object, the present invention provides a stretchable composite sheet having features described below.

The stretchable composite sheet includes: (a) a first nonwoven fabric; and (b) a net-shaped elastic sheet which is composed mainly of a thermoplastic elastic resin and whose fibrous or linear elements partially overlap with each other. The elastic sheet is bonded to the first nonwoven fabric at a plurality of locations away from each other.

The stretchable composite sheet having the above features has high breathability because the elastic sheet having high breathability is bonded to the nonwoven fabric.

Preferably, the stretchable composite sheet further includes (c) a second nonwoven fabric. The second nonwoven fabric is laminated to the elastic sheet and the first nonwoven fabric such that the elastic sheet is sandwiched between the first nonwoven fabric and the second nonwoven fabric. The second nonwoven fabric is bonded to at least either one of the elastic sheet and the first nonwoven fabric at a plurality of locations away from each other.

In this case, the stretchable composite sheet includes the elastic sheet sandwiched between the first nonwoven fabric and the second nonwoven fabric.

### Effects of the Invention

The present invention makes it possible to manufacture an elastic sheet having high breathability, manufacture a stretchable composite sheet having high breathability, and provide a stretchable composite sheet having high breathability.

### Brief Description of the Drawings

[FIG. 1] FIG. 1 is a schematic diagram illustrating an overall structure of a stretchable composite sheet manufacturing apparatus (First Embodiment).
[FIG. 2] FIG. 2 is a diagram illustrating an application pattern (First Embodiment).
[FIG. 3] FIG. 3 is a diagram illustrating an application pattern (First Embodiment).
[FIG. 4] FIG. 4(a) is a cross-sectional view of a stretchable composite sheet, with its elastic sheet in a stretched state. FIG. 4(b) is a cross-sectional view of the stretchable composite sheet, with its elastic sheet brought out of the stretched state (First Embodiment).
[FIG. 5] FIG. 5 is a schematic diagram illustrating an overall structure of a stretchable composite sheet manufacturing apparatus (Second Embodiment).
[FIG. 6] FIG. 6 is a diagram illustrating an application pattern (Second Embodiment).
[FIG. 7] FIG. 7 is a schematic diagram illustrating an overall structure of a stretchable composite sheet manufacturing apparatus (Third Embodiment).
[FIG. 8] FIG. 8 is a schematic diagram illustrating main components of the stretchable composite sheet manufacturing apparatus (Third Embodiment).
[FIG. 9] FIG. 9 is a schematic diagram illustrating a stretchable composite sheet manufacturing method (Conventional Example 1).

### Mode for Carrying out the Invention

Embodiments of the present invention will be described below with reference to the drawings.

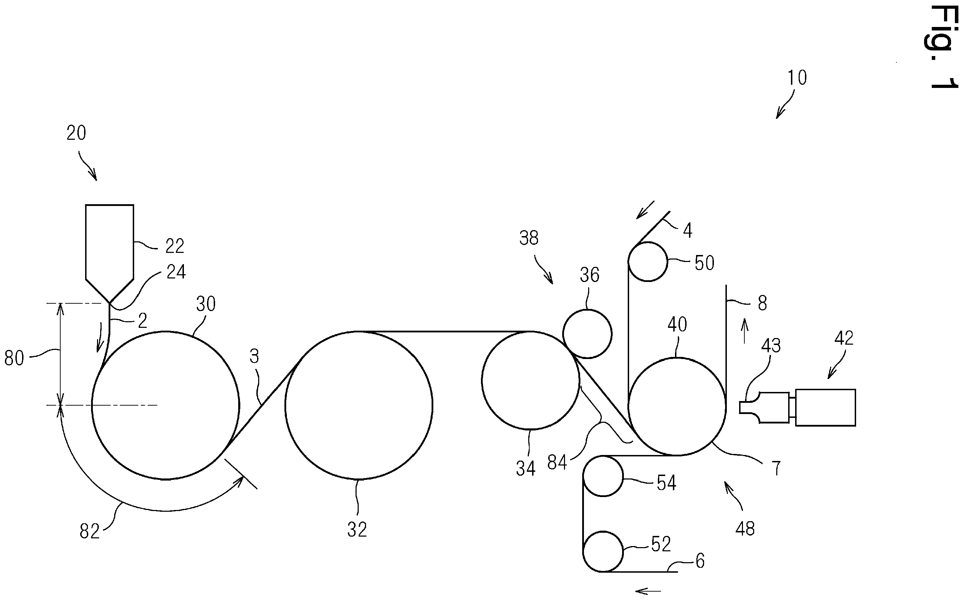

<First embodiment> An elastic sheet manufacturing method and a stretchable composite sheet manufacturing method according to first embodiment will be described with reference to FIG. 1 to FIG. 4.

FIG. 1 is a schematic diagram illustrating an overall structure of a stretchable composite sheet manufacturing apparatus 10. As illustrated in FIG. 1, the stretchable composite sheet manufacturing apparatus 10 includes an applicator 20, a cooling roller 30, a stretcher 38, a laminator 48, and a controller (not illustrated). The applicator 20 includes a discharger 22. The cooling roller 30 is a cooling member. The controller exercises control such that the discharger 22, the cooling roller 30, the stretcher 38, and the laminator 48 operate in conjunction with each other. The stretchable composite sheet manufacturing apparatus 10 is able to manufacture an elastic sheet 3 and a stretchable composite sheet 8.

The discharger 22 includes at least one discharge port 24. The discharger 22 discharges, from the discharge port 24, a heated and molten elastic resin material in a fibrous or linear form so as to form an intermediate 2 in a fibrous or linear form. The elastic resin material is composed mainly of a thermoplastic elastic resin. The elastic resin material is melted by being heated to a temperature higher than a temperature range in which the elastic resin material elastically deforms. The cooling roller 30, which is rotary, is disposed below the discharger 22.

The applicator 20 is configured to apply the intermediate 2 (which has been discharged from the discharge port 24 of the discharger 22) in a net form to the outer peripheral surface of the cooling roller 30 (which is rotary). Specifically, the applicator 20 applies the intermediates 2 to the outer peripheral surface of the cooling roller 30 such that the intermediates 2, each in a fibrous form or linear form, partially overlap with each other, resulting in a net form.

In one example, an elastic resin material may be discharged from the discharger 22 at intervals, or an elastic resin material may be discharged continuously in a linear form and cut by, for example, a jet of air in a first section 80 defined between the discharger 22 and the cooling roller 30, thus forming the intermediates 2 in a fibrous form. Depositing the intermediates 2 in a fibrous form on the cooling roller 30 such that the intermediates 2 are randomly oriented enables application of the fibrous intermediates 2 in a net form.

An elastic resin material may be continuously applied to the cooling roller 30, and then another elastic resin material may be applied thereto at intervals. In this case, laminating layers at intervals to a continuous layer makes it possible to easily form regions where stretching stress differs.

One example involves, when the intermediates 2 are each in a linear form, emitting a jet of hot air around the intermediates 2 in the first section 80 (which is defined between the discharger 22 and the cooling roller 30) so as to produce a spiral air current, and shaking or spirally turning the intermediates 2 by this air current, thus applying the intermediates 2 to the cooling roller 30 in a serpentine pattern or a spiral pattern. A technique that involves spiral pattern application is known in, for example, Japanese Patent No. 2916784. A technique that involves serpentine pattern application is known in, for example, Japanese Patent No. 4361646.

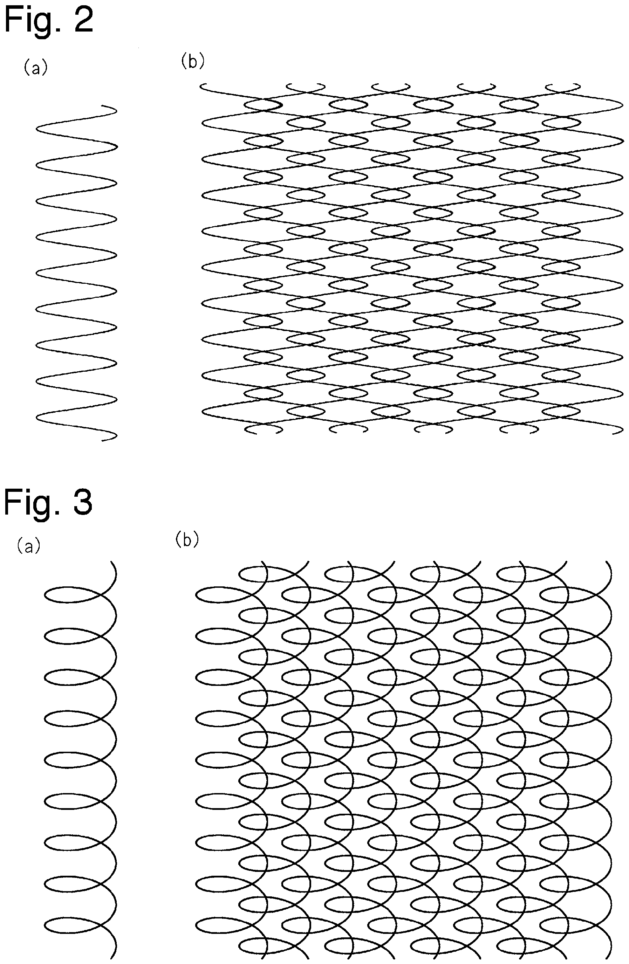

FIGS. 2 and 3 are diagrams schematically illustrating application patterns. The up-down direction in FIGS. 2 and 3 corresponds to a flow direction during manufacturing process. Using a discharger including a plurality of discharge ports or a plurality of dischargers each including a single discharge port, the elastic resin material (i.e., the intermediate 2) discharged from each discharge port is applied in a serpentine pattern illustrated in FIG. 2(a) or in a spiral pattern illustrated in FIG. 3 (a). As illustrated in FIG. 2(b) and FIG. 3(b), the elastic resin materials (i.e., the intermediates 2) discharged from the discharge ports are applied to the cooling member (i.e., the cooling roller 30) such that the elastic resin materials partially overlap with each other. In accordance with the features of the discharge ports, the characteristics of resulting application patterns may change. Examples of the characteristics include: the thickness of each application pattern (i.e., the amount of each elastic resin material to be discharged); the size of each application pattern; the cycle period of each application pattern; the type of each application pattern; and the degree of overlapping of adjacent application patterns.

The cooling roller 30 is internally provided with a flow passage (not illustrated) through which a coolant flows. In a second section 82 where the intermediates 2 are in contact with the outer peripheral surface of the cooling roller 30, the coolant cools the intermediates 2 to a temperature range in which the elastic resin materials (which constitute the intermediates 2) elastically deform, such that the intermediates 2 are solidified. The intermediates 2 (which have been applied in a net form to the outer peripheral surface of the rotating cooling roller 30) are thus changed into a net-shaped elastic sheet 3, whose fibrous or linear elements partially overlap with each other, in the second section 82. The elastic sheet 3 is then delivered out of the cooling roller 30.

The net-shaped elastic sheet 3 is delivered to a guide roller 32 and then to the stretcher 38. The stretcher 38 includes a delivery roller 34, a pinch roller 36, and a stretching roller 40. The elastic sheet 3 is sandwiched between the delivery roller 34 and the pinch roller 36 such that the elastic sheet 3 does not slip on the outer peripheral surface of the delivery roller 34. Rotation of the delivery roller 34 synchronizes with rotation of the cooling roller 30.

A circulating cooling member (e.g., an endless belt) may be used instead of the cooling roller 30.

The elastic sheet 3 is stretched in an elastic sheet flow direction (which is perpendicular to the width direction and thickness direction of the elastic sheet 3) in a third section 84 defined between an in-between space of the delivery roller 34 and the pinch roller 36 and the stretching roller 40. Specifically, the stretching roller 40 rotates at a peripheral speed higher than the peripheral speed of the delivery roller 34 so as to stretch the elastic sheet 3 by a predetermined factor. The elastic sheet 3 is thus being stretched along the outer peripheral surface of the stretching roller 40.

The laminator 48 supplies a first nonwoven fabric 4 in a continuous form to a guide roller 50 and then to the stretching roller 40. The first nonwoven fabric 4 is a first base material sheet. The laminator 48 supplies a second nonwoven fabric 6 in a continuous form to guide rollers 52 and 54 and then to the stretching roller 40. The second nonwoven fabric 6 is a second base material sheet. The elastic sheet 3 stretched along the stretching roller 40 is sandwiched between the first nonwoven fabric 4 and the second nonwoven fabric 6. This provides a laminated body 7 in which the elastic sheet 3, the first nonwoven fabric 4, and the second nonwoven fabric 6 are laminated to each other.

The laminated body 7 moves in accordance with the rotation of the stretching roller 40 and passes through a space between the stretching roller 40 and a horn 43 of an ultrasonic bonder 42. The horn 43 moves close to and away from the stretching roller 40, such that the elastic sheet 3 is ultrasonically bonded to the first and second nonwoven fabrics 4 and 6 when the laminated body 7 is located between the stretching roller 40 and the horn 43. Portions of the first and second nonwoven fabrics 4 and 6 directly facing each other are ultrasonically bonded to each other, with no elastic sheet 3 interposed therebetween.

The method thus manufactures the stretchable composite sheet 8 in which the elastic sheet 3 is boded at intervals to the first and second nonwoven fabrics 4 and 6. The stretchable composite sheet 8 is then delivered out of the laminator 48.

Specifically, the stretching roller 40 is provided on its outer peripheral surface with protrusions (not illustrated) spaced from each other and thus functions as an anvil. A portion of the laminated body 7 located between the protrusions of the stretching roller 40 and the horn 43 is subjected to ultrasonic bonding. Such ultrasonic bonding makes it possible to bond the elastic sheet 3 to the first and second nonwoven fabrics 4 and 6 with ease and precision, and makes it possible to bond the first nonwoven fabric 4 to the second nonwoven fabric 6, with no elastic sheet 3 interposed therebetween, with ease and precision. The elastic sheet 3 of the laminated body 7 may naturally be bonded to the first and second nonwoven fabrics 4 and 6 by thermal welding (such as heat sealing) or an embossing process, for example.

The stretching roller 40 serves not only as a roller included in the stretcher 38 but also as a roller included in the laminator 48. The stretchable composite sheet manufacturing apparatus 10 is thus simplified in structure. Alternatively, the stretcher 38 and the laminator 48 may include different rollers.

The stretchable composite sheet 8 manufactured by the stretchable composite sheet manufacturing apparatus 10 includes: the first and second nonwoven fabrics 4 and 6; and the net-shaped elastic sheet 3 which is composed mainly of a thermoplastic elastic resin and whose fibrous or linear elements partially overlap with each other. The elastic sheet 3 is bonded to the first nonwoven fabric 4 at a plurality of locations away from each other. The second nonwoven fabric 6 is laminated to the elastic sheet 3 and the first nonwoven fabric 4 such that the elastic sheet 3 is sandwiched between the first nonwoven fabric 4 and the second nonwoven fabric 6. The second nonwoven fabric 6 is bonded to at least either one of the elastic sheet 3 and the first nonwoven fabric 4 at a plurality of locations away from each other.

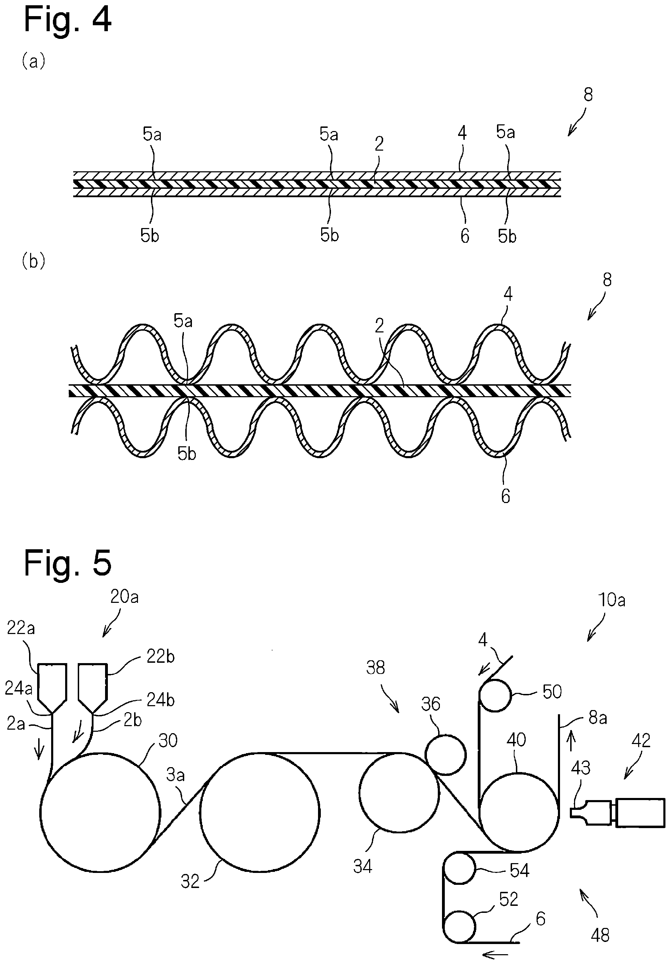

FIG. 4(a) is a cross-sectional view of the stretchable composite sheet 8, with the elastic sheet 3 in a stretched state. As illustrated in FIG. 4(a), the stretched elastic sheet 3 is bonded at intervals to the first and second nonwoven fabrics 4 and 6, each having a planar shape, at a plurality of junctions 5a and 5b.

FIG. 4(b) is a cross-sectional view of the stretchable composite sheet, with the elastic sheet 3 brought out of the stretched state. As illustrated in FIG. 4(b), bringing the elastic sheet 3 out of the stretched state reduces the spaces between the junctions 5a adjacent to each other and the spaces between the junctions 5b adjacent to each other, and bends the first and second nonwoven fabrics 4 and 6, making the surfaces of the first and second nonwoven fabrics 4 and 6 uneven.

A thermoplastic elastic resin to be used as a material for the elastic sheet 3 is preferably a thermoplastic resin that exhibits rubber elasticity at room temperature. One example may involve selecting a suitable type of thermoplastic resin from thermoplastic elastomers specified and classified in JIS K 6418: 2007 (ISO 18064: 2003). Another example may involve using a thermoplastic elastomer whose hard segment has a glass transition temperature of about 100°C to about 200°C and whose soft segment has a glass transition temperature of -70°C to -10°C.

Examples of a thermoplastic elastic resin usable as a material for the elastic sheet 3 include: an olefinic elastomer, such as "VERSIFY" (registered trademark) produced by the Dow Chemical Company; a propylene elastomer, such as "Vistamaxx" (registered trademark) produced by Exxon Mobil Corporation; and a styrene elastomer, such as "Quintac" (registered trademark) produced by Zeon Corporation.

The laminator 48 of the stretchable composite sheet manufacturing apparatus 10 may be configured to supply the first nonwoven fabric 4 instead of supplying both of the first and second nonwoven fabrics 4 and 6 and to laminate and bond the elastic sheet 3 to the first nonwoven fabric 4. The stretchable composite sheet manufactured in this case includes: the first nonwoven fabric 4; and the net-shaped elastic sheet 3 which is composed mainly of a thermoplastic elastic resin and whose fibrous or linear elements partially overlap with each other. The elastic sheet 3 is bonded to the first nonwoven fabric 4 at a plurality of locations away from each other.

The laminator 48 may be configured to supply only the second nonwoven fabric 6 and laminate the elastic sheet 3 to the second nonwoven fabric 6 such that the elastic sheet 3 is thermally welded to the second nonwoven fabric 6.

The stretchable composite sheet manufacturing apparatus 10 may be configured to include no stretcher 38 and stretch the elastic sheet 3 together with the nonwoven fabrics 4 and 6 after the laminated body 7 has undergone boding. In this case, fabrics highly stretchable in the width direction (which is perpendicular to the flow direction and the thickness direction), such as spun-laced non-woven fabrics, may be used as the first and second nonwoven fabrics 4 and 6. This enables manufacture of a stretchable composite sheet highly stretchable not only in the width direction but also in the flow direction. After the elastic sheet 3 is laminated and bonded to either one of the first and second nonwoven fabrics 4 and 6, the elastic sheet 3 may be stretched together with the one of the nonwoven fabrics 4 and 6.

The elastic sheet manufacturing method will be described below with reference to FIG. 1. First, the method includes a first step involving heating an elastic resin material, which is composed mainly of a thermoplastic elastic resin, to a temperature higher than a temperature range in which the elastic resin material elastically deforms, such that the elastic resin material is molten. The method then includes a second step involving discharging the molten elastic resin material in a fibrous or linear form from the discharge port 24. The method subsequently includes a third step involving applying the elastic resin material (i.e., the intermediate 2), which has been discharged from the discharge port 24, in a net form to the cooling member (i.e., the cooling roller 30), and cooling the applied elastic resin material by the cooling member (i.e., the cooling roller 30) to the temperature range in which the elastic resin material elastically deforms, such that the elastic resin material is solidified so as to form the elastic sheet 3. The method thus provides the net-shaped elastic sheet 3, enabling manufacture of the elastic sheet 3 having high breathability. The method also enables manufacture of the elastic sheet 3 stretchable in all directions.

The second step may involve discharging the elastic resin material in a linear form, and the third step may involve applying the elastic resin material, which has been discharged in a linear form, to the cooling member in a serpentine pattern or in a spiral pattern. In this case, the elastic resin material is easily applied in a net form.

The second step may involve discharging the elastic resin material from each of a plurality of discharge ports (e.g., a plurality of discharge ports arranged in a direction perpendicular to the plane of FIG. 1), and the third step may involve applying the elastic resin materials (i.e., the intermediates 2), which have been discharged from the discharge ports, to the cooling member (i.e., the cooling roller 30) such that the elastic resin materials partially overlap with each other. In this case, the method enables manufacture of the net-shaped elastic sheet having a large width.

The second step may involve discharging the elastic resin materials from the discharge ports such that each amount of elastic resin materials discharged from one or plural of the discharge ports differs from each amount of elastic resin materials discharged from the other of the discharge ports. In this case, the method enables manufacture of the elastic sheet 3 having different stretching characteristics at different positions in the width direction.

The second step may involve periodically changing the amount or amounts of elastic resin material or materials to be discharged. In this case, the method enables manufacture of the elastic sheet 3 having different stretching characteristics at different positions in the flow direction (which is perpendicular to the width direction and the thickness direction).

The stretchable composite sheet manufacturing method will be described below with reference to FIG. 1.

Laminating and bonding the elastic sheet 3 (which has been manufactured by the elastic sheet manufacturing method described above) to the first nonwoven fabric 4 enables manufacture of a stretchable composite sheet in which the elastic sheet 3 having high breathability is bonded to the first nonwoven fabric 4 and which thus has high breathability.

The second nonwoven fabric 6 may be laminated to the elastic sheet 3 and the first nonwoven fabric 4 such that the elastic sheet 3 is sandwiched between the first nonwoven fabric 4 and the second nonwoven fabric 6, and may be bonded to at least either one of the elastic sheet 3 and the first nonwoven fabric 4. In this case, the method enables manufacture of the stretchable composite sheet 8 in which the elastic sheet 3 is sandwiched between the first nonwoven fabric 4 and the second nonwoven fabric 6.

The elastic sheet 3 may be ultrasonically bonded to the first nonwoven fabric 4 at a plurality of locations away from each other. When the elastic resin material(s) for the elastic sheet 3 has or have adhesion properties similar to those of a hot-melt adhesive, the elastic sheet 3 is easily bondable to the first nonwoven fabric 4. This facilitates both of bonding between the elastic sheet 3 and the first nonwoven fabric 4 and bonding between the first nonwoven fabric 4 and the second nonwoven fabric 6.

The elastic sheet 3 may be stretched, and the elastic sheet 3 in a stretched state may be laminated and bonded to the first nonwoven fabric 4. In this case, the method is able to provide the stretchable composite sheet 8 in which the first nonwoven fabric 4 is made uneven and the unevenness of the first nonwoven fabric 4 is smoothed out by stretching the stretchable composite sheet 8.

The elastic sheet 3 may be stretched together with the first nonwoven fabric 4 after the elastic sheet 3 is laminated and bonded to the first nonwoven fabric 4. In this case, the method is able to provide the stretchable composite sheet in which the surface of the first nonwoven fabric 4 is kept substantially even after the elastic sheet 3 is brought out of the stretched state.

The elastic sheet 3 may be laminated and bonded to the first nonwoven fabric 4 stretchable at least in one direction, such that the one direction in which the first nonwoven fabric 4 is stretchable is perpendicular to a direction in which the elastic sheet 3 is to be stretched. In this case, the method is able to provide the stretchable composite sheet stretchable in all directions.

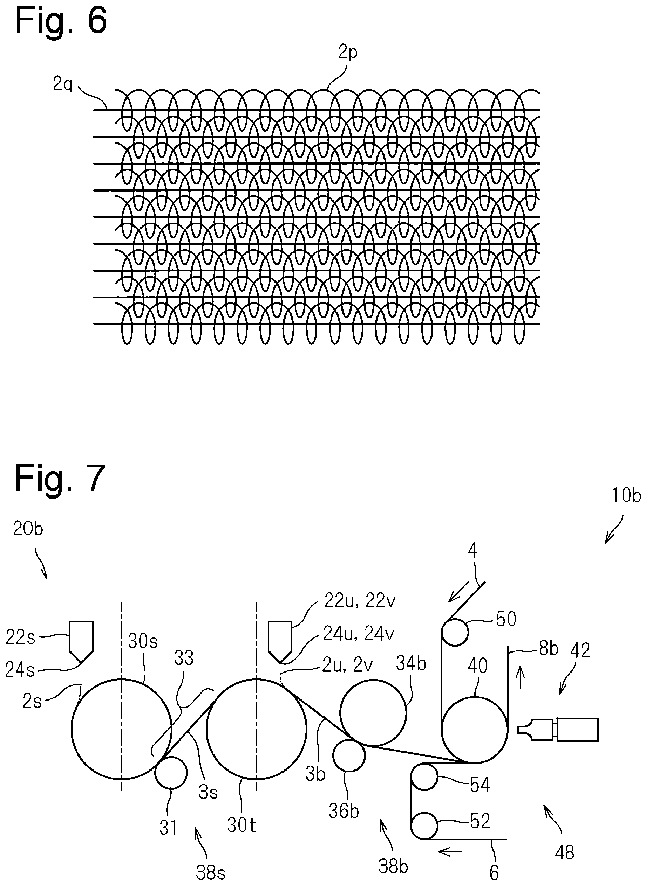

<Second embodiment> Second embodiment will be described with reference to FIGS. 5 and 6. second embodiment is substantially similar to first embodiment. In the following description, elements similar to those of first embodiment will be identified by the same reference characters. The following description focuses on differences between second embodiment and first embodiment.

FIG. 5 is a schematic diagram illustrating an overall structure of a stretchable composite sheet manufacturing apparatus 10a according to second embodiment. As illustrated in FIG. 5, the stretchable composite sheet manufacturing apparatus 10a includes an applicator 20a, a cooling roller 30, a stretcher 38, and a laminator 48. The applicator 20a includes first and second dischargers 22a and 22b. The cooling roller 30 is a cooling member disposed below the first and second dischargers 22a and 22b. The cooling roller 30, the stretcher 38, and the laminator 48 are similar in structure to those of first embodiment.

Similarly to the discharger 22 according to first embodiment, the first discharger 22a includes a first discharge port 24a to discharge a first elastic resin material in a fibrous or linear form. The first elastic resin material is composed mainly of a first thermoplastic elastic resin. The first elastic resin material is heated to a temperature higher than a first temperature range in which the first elastic resin material elastically deforms, such that the first elastic resin material is molten.

The second discharger 22b includes a plurality of second discharge ports 24b to discharge second elastic resin materials in a linear form. The second elastic resin materials are each composed mainly of a second thermoplastic elastic resin. The second elastic resin materials are heated to a temperature higher than a second temperature range in which the second elastic resin materials elastically deform, such that the second elastic resin materials are molten.

The first and second thermoplastic elastic resins may be similar to those used in first embodiment. The first and second thermoplastic elastic resins may be identical or different from each other. When the first and second thermoplastic elastic resins are identical, the first and second elastic resin materials may be identical or different from each other. When the first and second elastic resin materials are identical, the first and second elastic resin materials may be heated and molten in the same manner.

FIG. 6 is a diagram schematically illustrating application patterns. The right-left direction in FIG. 6 corresponds to the flow direction during manufacturing process. As illustrated in FIG. 6, each first elastic resin material is applied in a first application pattern 2p in a net form, and each second elastic resin material is applied in a second application pattern 2q in a linear form.

Specifically, the applicator 20a applies the first elastic resin materials (i.e., intermediates 2a), which have been discharged from the first discharge port 24a of the first discharger 22a, in a net form to the cooling roller 30, and applies the second elastic resin materials (i.e., intermediates 2b), which have been discharged from the second discharge ports 24b of the second discharger 22b, in a linear form to the cooling roller 30 such that the second elastic resin materials (i.e., the intermediates 2b) are in parallel with each other and overlap with the first elastic resin materials (i.e., the intermediates 2a) applied to the cooling roller 30.

Suppose that an elastic sheet is to be formed only in a spiral pattern or a serpentine pattern. In this case, changes in parameters, such as the amount of each elastic resin material (e.g., the thickness of each elastic resin material and/or the number of elastic resin materials) and the size and cycle of each application pattern, result in corresponding changes in stretching stress in both of the flow direction (which is perpendicular to the width direction and thickness direction) and the width direction. This makes it difficult to adjust stretching stress in the flow direction and the width direction.

A combination of the first application pattern 2p (which is in a net form) and the second application pattern 2q (which is in a linear form) extending in the flow direction makes it possible to adjust stretching stress mainly in the width direction by changing parameters, such as the amount of each first elastic resin material (e.g., the thickness of each first elastic resin material and/or the number of first elastic resin materials) to be applied in the first application pattern 2p and the size and cycle of each application pattern. This combination also makes it possible to adjust stretching stress mainly in the flow direction by changing the amount of each second elastic resin material (e.g., the thickness of each second elastic resin material and/or the number of second elastic resin materials) to be applied in the second application pattern 2q. Consequently, this combination facilitates adjustment of stretching stress in the flow direction and the width direction.

The cooling roller 30 cools the first and second elastic resin materials (which are applied to the cooling roller 30) to a temperature range in which the first and second elastic resin materials elastically deform, such that the first and second elastic resin materials are solidified so as to form an elastic sheet 3a. Similarly to first embodiment, the elastic sheet 3a is stretched by the stretcher 38 and then laminated and bonded to the first and second nonwoven fabrics 4 and 6 by the laminator 48. This provides a stretchable composite sheet 8a.

An elastic sheet manufacturing method according to second embodiment will be described below.

First, the method includes a first step involving heating a first elastic resin material (which is composed mainly of a first thermoplastic elastic resin) to a temperature higher than a first temperature range in which the first elastic resin material elastically deforms, such that the first elastic resin material is molten. The first step includes a first sub-step involving heating a second elastic resin material (which is composed mainly of a second thermoplastic elastic resin) to a temperature higher than a second temperature range in which the second elastic resin material elastically deforms, such that the second elastic resin material is molten.

The method then includes a second step involving discharging the molten first elastic resin material in a fibrous or linear form from a first discharge port. The second step includes a second sub-step involving discharging the molten second elastic resin material in a linear form from each of a plurality of second discharge ports.

The method subsequently includes a third step involving: applying the first elastic resin material (which has been discharged from the first discharge port) in a net form to the cooling member (i.e., the cooling roller 30); and cooling the applied first elastic resin material to the first temperature range by the cooling member (i.e., the cooling roller 30) such that the first elastic resin material is solidified. The third step includes a third sub-step involving: applying the second elastic resin materials (which have been discharged from the second discharge ports) in a linear form to the cooling member (i.e., the cooling roller 30) such that the second elastic resin materials are in parallel with each other and overlap with the first elastic resin material applied to the cooling member (i.e., the cooling roller 30); and cooling the applied second elastic resin materials to the second temperature range by the cooling member (i.e., the cooling roller 30) such that the second elastic resin materials are solidified.

The method is able to provide the net-shaped elastic sheet 3a including the first and second elastic resin materials cooled and solidified and thus enables manufacture of the elastic sheet 3a having high breathability. The method also enables manufacture of the elastic sheet 3a stretchable in all directions.

Changing the amount of first elastic resin material (e.g., the thickness of each first elastic resin material and/or the number of first elastic resin materials) or an application pattern thereof makes it possible to adjust stretching stress mainly in the width direction of the elastic sheet. Changing the amounts of second elastic resin materials (e.g., the thickness of each second elastic resin material and/or the number of second elastic resin materials) makes it possible to adjust stretching stress mainly in the flow direction of the elastic sheet. Consequently, the method facilitates adjustment of stretching stress in the flow direction and the width direction.

The first and second thermoplastic elastic resins may be identical or different from each other. When the first and second thermoplastic elastic resins are identical, the first and second elastic resin materials may be identical or different from each other.

<Third embodiment> Third embodiment will be described with reference to FIGS. 7 and 8. third embodiment is substantially similar to Embodiments 1 and 2. In the following description, elements similar to those of Embodiments 1 and 2 will be identified by the same reference characters. The following description focuses on differences between third embodiment and Embodiments 1 and 2.

FIG. 7 is a schematic diagram illustrating an overall structure of a stretchable composite sheet manufacturing apparatus 10b according to third embodiment. FIG. 8 is a schematic diagram illustrating main components of the stretchable composite sheet manufacturing apparatus 10b. As illustrated in FIGS. 7 and 8, the stretchable composite sheet manufacturing apparatus 10b includes an applicator 20b, a first cooling roller 30s, a second cooling roller 30t, a stretcher 38b, and a laminator 48. The applicator 20b includes a first discharger 22s and second dischargers 22u and 22v. The first cooling roller 30s is a cooling member disposed below the first discharger 22s. The second cooling roller 30t is an additional cooling member disposed below the second dischargers 22u and 22v. The second cooling roller 30t is disposed downstream of the first cooling roller 30s. The laminator 48 is similar in structure to the laminator 48 according to first embodiment.

The first discharger 22s includes a first discharge port 24s to discharge a first elastic resin material 2s in a fibrous or linear form. The first elastic resin material 2s is composed mainly of a first thermoplastic elastic resin. The first elastic resin material 2s is heated to a temperature higher than a first temperature range in which the first elastic resin material 2s elastically deforms, such that the first elastic resin material is molten. The applicator 20b applies the first elastic resin material 2s (which has been discharged from the first discharge port 24s) in a net form to the outer peripheral surface of the first cooling roller 30s. The first cooling roller 30s cools the first elastic resin material (which is applied to the first cooling roller 30s) to the first temperature range in which the first elastic resin material elastically deforms, such that the first elastic resin material is solidified. This forms a net-shaped elastic sheet 3s. The elastic sheet 3s delivered out of the first cooling roller 30s passes along the second cooling roller 30t.

The second dischargers 22u and 22v respectively include second discharge ports 24u and 24v to discharge second elastic resin materials 2u and 2v in a linear or fibrous form. The second elastic resin materials 2u and 2v are each composed mainly of a second thermoplastic elastic resin. The second elastic resin materials 2u and 2v are heated to a temperature higher than a second temperature range in which the second elastic resin materials 2u and 2v elastically deform, such that the second elastic resin materials 2u and 2v are molten. The applicator 20b applies the second elastic resin materials 2u and 2v (which are respectively discharged from the second discharge ports 24u and 24v) in a linear or net form to the outer peripheral surface of the second cooling roller 30t. The second cooling roller 30t cools the second elastic resin materials (which are applied to the second cooling roller 30t) to the second temperature range in which the second elastic resin materials elastically deform, such that the second elastic resin materials are solidified. Alternatively, the number of second dischargers may be one or may be three or more. When two or more second dischargers are provided, the second dischargers may be different in structure or may be similar in structure.

A region where the first elastic resin material discharged from the first discharge port 24s is to be applied and a region where the second elastic resin materials discharged from the second discharge ports 24u and 24v are to be applied may overlap with each other, or may be separate regions. When the region where the first elastic resin material is to be applied and the region where the second elastic resin materials are to be applied overlap with each other, the method enables manufacture of a sheet-shaped elastic member 3b (i.e., an elastic sheet 3b) including the first and second elastic resin materials. When these regions do not overlap with each other, the method enables manufacture of the elastic member 3b including the net-shaped first elastic resin material (i.e., the elastic sheet 3s) and the linear or net-shaped second elastic resin materials, with the first elastic resin material separated from the second elastic resin materials.

When the region where the first elastic resin material is to be applied and the region where the second elastic resin materials are to be applied overlap with each other, the second elastic resin materials discharged from the second discharge ports 24u and 24v may be applied onto the elastic sheet 3s made of the first elastic resin material, or may be applied to the second cooling roller 30t, in which case the elastic sheet 3s made of the first elastic resin material may be placed on the second elastic resin materials.

An additional stretcher 38s may be provided so as to stretch the elastic sheet 3s made of the first elastic resin material. In this case, a pinch roller 31 is disposed to face the first cooling roller 30s, and the elastic sheet 3s is sandwiched between the first cooling roller 30s and the pinch roller 31 such that the elastic sheet 3s does not slip on the outer peripheral surface of the first cooling roller 30s. The second cooling roller 30t rotates at a peripheral speed higher than the peripheral speed of the first cooling roller 30s. The elastic sheet 3s delivered out of the first cooling roller 30s is thus stretched by a predetermined factor in a section 33 defined between the first cooling roller 30s and the second cooling roller 30t.

Similarly to the stretcher 38 according to first embodiment, the stretcher 38b includes a delivery roller 34b, a pinch roller 36b, and a stretching roller 40. Similarly to first embodiment, the elastic member 3b (which includes the elastic sheet 3s) delivered out of the second cooling roller 30t is stretched by the stretcher 38b and is then laminated and bonded to the first and second nonwoven fabrics 4 and 6 by the laminator 48. This provides a stretchable composite sheet 8b.

Similarly to second embodiment, the use of the first and second elastic resin materials facilitates adjustment of stretching stress in the flow direction and the width direction. Because the first and second elastic resin materials are separately cooled and solidified, third embodiment is more likely to make a difference in stretching rate than second embodiment in which the first and second elastic resin materials are simultaneously cooled and solidified. Providing the additional stretcher 38s facilitates increasing a difference in stretching rate.

A method for manufacturing the elastic sheet 3b according to third embodiment will be described below.

First, the method includes a first step involving heating a first elastic resin material (which is composed mainly of a first thermoplastic elastic resin) to a temperature higher than a first temperature range in which the first elastic resin material elastically deforms, such that the first elastic resin material is molten. The first step includes a first sub-step involving heating second elastic resin materials (which are each composed mainly of a second thermoplastic elastic resin) to a temperature higher than a second temperature range in which the second elastic resin materials elastically deform, such that the second elastic resin materials are molten.

The method then includes a second step involving discharging the molten first elastic resin material in a fibrous or linear form from the first discharge port 24s. The second step includes a second sub-step involving discharging the molten second elastic resin materials in a linear or fibrous form from the second discharge ports 24u and 24v.

The method subsequently includes a third step involving: applying the first elastic resin material (which has been discharged from the first discharge port 24s) in a net form to the cooling member (i.e., the first cooling roller 30s); and cooling the applied first elastic resin material to the first temperature range by the cooling member (i.e., the first cooling roller 30s) such that the first elastic resin material is solidified. The third step includes a third sub-step involving: delivering the first elastic resin material (i. e., the elastic sheet 3s), which has been cooled to the first temperature range such that the first elastic resin material is solidified, out of the cooling member (i.e., the first cooling roller 30s); causing the first elastic resin material to pass along the additional cooling member (i.e., the second cooling roller 30t); applying the second elastic resin materials (which have been discharged from the second discharge ports 24u and 24v) in a linear or net form to the additional cooling member (i.e., the second cooling roller 30t) such that the second elastic resin materials overlap with a portion of the first elastic resin material (i.e., the elastic sheet 3s) extending along the additional cooling member (i.e., the second cooling roller 30t); and cooling the applied second elastic resin materials to the second temperature range by the additional cooling member (i.e., the second cooling roller 30t) such that the second elastic resin materials are solidified.

The method enables manufacture of the sheet-shaped elastic member 3b (i.e., the elastic sheet 3b) including the first and second elastic resin materials. The elastic sheet 3b having high breathability is manufacturable because the first elastic resin material applied in a net form and the second elastic resin materials applied in a linear or net form overlap with each other. The method also enables manufacture of the elastic sheet 3b stretchable in all directions.

Similarly to second embodiment, the use of the first and second elastic resin materials facilitates adjustment of stretching stress in the flow direction and the width direction of the elastic sheet 3b. Because the first and second elastic resin materials are separately cooled and solidified, third embodiment is more likely to make a difference in stretching rate than second embodiment in which the first and second elastic resin materials are simultaneously cooled and solidified.

The first and second thermoplastic elastic resins may be identical or different from each other. When the first and second thermoplastic elastic resins are identical, the first and second elastic resin materials may be identical or different from each other.

Manufacture of the stretchable composite sheet 8b may involve causing the first elastic resin material (which is applied in a net form) and the second elastic resin materials (which are applied in a linear or net form) to overlap with each other, or may involve preventing the first elastic resin material and the second elastic resin materials from overlapping with each other. In an alternative embodiment, the second elastic resin materials discharged in a linear or fibrous form from the second discharge ports 24u and 24v may be applied to the additional cooling member (i.e., the second cooling roller 30t) such that the second elastic resin materials do not overlap with a portion of the first elastic resin material passing along the additional cooling member (i.e., the second cooling roller 30t). Also in this case, a stretchable composite sheet including an elastic sheet having high breathability is manufacturable.

<Summary> As described above, an elastic sheet having high breathability is manufacturable. A stretchable composite sheet including an elastic sheet having high breathability is manufacturable.

The present invention is not limited to the foregoing embodiments but may be practiced, with various modifications made thereto.

### Description of Reference Numerals

- 3, 3a: elastic sheet
- 4: first nonwoven fabric
- 6: second nonwoven fabric
- 8, 8a, 8b: stretchable composite sheet
- 10, 10a, 10b: stretchable composite sheet manufacturing apparatus
- 20, 20a, 20b: applicator
- 22, 22a, 22b, 22s, 22u, 22v: discharger
- 24, 24a, 24b, 24s, 24u, 24v: discharge port
- 30: cooling roller (cooling member)
- 30s: cooling roller (cooling member)
- 30t: cooling roller (additional cooling member)
- 38, 38b, 38s: stretcher
- 48: laminator

## Claims

1. An elastic sheet manufacturing method comprising:
a first step involving heating a first elastic resin material to a temperature higher than a first temperature range in which the first elastic resin material elastically deforms, such that the first elastic resin material is molten, the first elastic resin material being composed mainly of a first thermoplastic elastic resin;
a second step involving discharging the molten first elastic resin material in a fibrous or linear form from a first discharge port; and
a third step involving applying the first elastic resin material, which has been discharged from the first discharge port, in a net form to a cooling member, and cooling the applied first elastic resin material to the first temperature range by the cooling member such that the first elastic resin material is solidified.

2. The elastic sheet manufacturing method according to claim 1, wherein
the second step involves discharging the first elastic resin material in a linear form, and
the third step involves applying the first elastic resin material, which has been discharged in the linear form, in a serpentine pattern or a spiral pattern to the cooling member.

3. The elastic sheet manufacturing method according to claim 1 or 2, wherein
the second step involves discharging the first elastic resin material from each of a plurality of the first discharge ports, and
the third step involves applying the first elastic resin materials, which have been discharged from the first discharge ports, to the cooling member such that the first elastic resin materials partially overlap with each other.

4. The elastic sheet manufacturing method according to claim 3, wherein
the second step involves discharging the first elastic resin materials from the first discharge ports such that each amount of the first elastic resin materials discharged from one or plural of the first discharge ports differs from each amount of the first elastic resin materials discharged from the other of the first discharge ports.

5. The elastic sheet manufacturing method according to any one of claims 1 to 4, wherein
the second step involves periodically changing an amount or amounts of the first elastic resin material or materials to be discharged.

6. The elastic sheet manufacturing method according to any one of claims 1 to 5, wherein
the first step includes a first sub-step involving heating second elastic resin materials, each composed mainly of a second thermoplastic elastic resin, to a temperature higher than a second temperature range in which the second elastic resin materials elastically deform, such that the second elastic resin materials are molten,
the second step includes a second sub-step involving discharging the molten second elastic resin materials in a linear form from a plurality of second discharge ports, and
the third step includes a third sub-step involving
applying the second elastic resin materials, which have been discharged from the second discharge ports, in a linear form to the cooling member such that the second elastic resin materials are in parallel with each other and overlap with the first elastic resin material or materials_applied to the cooling member, and
cooling the applied second elastic resin materials to the second temperature range by the cooling member such that the second elastic resin materials are solidified.

7. The elastic sheet manufacturing method according to any one of claims 1 to 5, wherein
the first step includes a first sub-step involving heating a second elastic resin material, which is composed mainly of a second thermoplastic elastic resin, to a temperature higher than a second temperature range in which the second elastic resin material elastically deforms, such that the second elastic resin material is molten,
the second step includes a second sub-step involving discharging the molten second elastic resin material in a linear or fibrous form from a second discharge port, and
the third step includes a third sub-step involving
delivering the first elastic resin material or materials, which has or have been cooled to the first temperature range such that the first elastic resin material or materials is or are solidified, out of the cooling member,
causing the first elastic resin material or materials to pass along an additional cooling member,
applying the second elastic resin material, which has been discharged from the second discharge port, in a linear or net form to the additional cooling member such that the second elastic resin material overlaps with a portion or portions of the first elastic resin material or materials extending along the additional cooling member, and
cooling the applied second elastic resin material to the second temperature range by the additional cooling member such that the second elastic resin material is solidified.

8. A stretchable composite sheet manufacturing method comprising laminating and bonding an elastic sheet to a first nonwoven fabric, the elastic sheet being manufactured by any one of the methods according to claims 1 to 7.

9. A stretchable composite sheet manufacturing method comprising laminating and bonding an elastic sheet to a first nonwoven fabric, the elastic sheet being manufactured by any one of the elastic sheet manufacturing methods according to any one of claims 1 to 5, wherein
the first step includes a first sub-step involving heating a second elastic resin material, which is composed mainly of a second thermoplastic elastic resin, to a temperature higher than a second temperature range in which the second elastic resin material elastically deforms, such that the second elastic resin material is molten,
the second step includes a second sub-step involving discharging the molten second elastic resin material in a linear or fibrous form from a second discharge port, and
the third step includes a third sub-step involving
delivering the first elastic resin material or materials, which has or have been cooled to the first temperature range such that the first elastic resin material or materials is or are solidified, out of the cooling member,
causing the first elastic resin material or materials to pass along an additional cooling member,
applying the second elastic resin material, which has been discharged from the second discharge port, in a linear or net form to the additional cooling member, and
cooling the applied second elastic resin material to the second temperature range by the additional cooling member such that the second elastic resin material is solidified.

10. The stretchable composite sheet manufacturing method according to claim 8 or 9, comprising laminating a second nonwoven fabric to the elastic sheet and the first nonwoven fabric such that the elastic sheet is sandwiched between the first nonwoven fabric and the second nonwoven fabric, and bonding the second nonwoven fabric to at least either one of the elastic sheet and the first nonwoven fabric.

11. The stretchable composite sheet manufacturing method according to claim 8 or 9, comprising ultrasonically bonding the elastic sheet to the first nonwoven fabric at a plurality of locations away from each other.

12. The stretchable composite sheet manufacturing method according to claim 8 or 9, comprising stretching the elastic sheet, and laminating and bonding the elastic sheet in a stretched state to the first nonwoven fabric.

13. The stretchable composite sheet manufacturing method according to claim 8 or 9, comprising laminating and bonding the elastic sheet to the first nonwoven fabric, and then stretching the elastic sheet together with the first nonwoven fabric.

14. The stretchable composite sheet manufacturing method according to claim 12 or 13, comprising laminating and bonding the elastic sheet to the first nonwoven fabric stretchable in at least one direction, such that the one direction in which the first nonwoven fabric is stretchable is perpendicular to a direction in which the elastic sheet is to be stretched.

15. An elastic sheet manufacturing apparatus comprising:
an applicator including a first discharger; and
a cooling member disposed below the first discharger, wherein
the first discharger includes a first discharge port to discharge a first elastic resin material in a fibrous or linear form, the first elastic resin material being composed mainly of a first thermoplastic elastic resin, the first elastic resin material being heated to a temperature higher than a first temperature range in which the first elastic resin material elastically deforms, such that the first elastic resin material is molten,
the applicator applies the first elastic resin material, which has been discharged from the first discharge port, in a net form to the cooling member, and
the cooling member cools the first elastic resin material, which has been applied to the cooling member, to the first temperature range such that the first elastic resin material is solidified.

16. The elastic sheet manufacturing apparatus according to claim 15, wherein
the applicator includes a second discharger,
the cooling member is disposed below the second discharger,
the second discharger includes a plurality of second discharge ports to discharge second elastic resin materials in a linear form, the second elastic resin materials each being composed mainly of a second thermoplastic elastic resin, the second elastic resin materials being heated to a temperature higher than a second temperature range in which the second elastic resin materials elastically deform, such that the second elastic resin materials are molten,
the applicator applies the second elastic resin materials, which have been discharged from the second discharge ports, in a linear form to the cooling member such that the second elastic resin materials are in parallel with each other and overlap with the first elastic resin material applied to the cooling member, and
the cooling member cools the second elastic resin materials, which have been applied to the cooling member, to the second temperature range such that the second elastic resin materials are solidified.

17. The elastic sheet manufacturing apparatus according to claim 15, further comprising an additional cooling member, wherein
the first elastic resin material, which has been applied to the cooling member and cooled to the first temperature range such that the first elastic resin material is solidified, is delivered out of the cooling member and is then passed along the additional cooling member,
the applicator includes a second discharger,
the additional cooling member is disposed below the second discharger,
the second discharger includes a second discharge port to discharge a second elastic resin material in a linear or fibrous form, the second elastic resin material being composed mainly of a second thermoplastic elastic resin, the second elastic resin material being heated to a temperature higher than a second temperature range in which the second elastic resin material elastically deforms, such that the second elastic resin material is molten,
the applicator applies the second elastic resin material, which has been discharged from the second discharge port, in a linear or net form to the additional cooling member such that the second elastic resin material overlaps with a portion of the first elastic resin material extending along the additional cooling member, and
the additional cooling member cools the second elastic resin material, which has been applied to the additional cooling member, to the second temperature range such that the second elastic resin material is solidified.

18. A stretchable composite sheet manufacturing apparatus comprising:
an applicator including a first discharger;
a cooling member disposed below the first discharger; and
a laminator, wherein
the first discharger includes a first discharge port to discharge a first elastic resin material in a fibrous or linear form, the first elastic resin material being composed mainly of a first thermoplastic elastic resin, the first elastic resin material being heated to a temperature higher than a first temperature range in which the first elastic resin material elastically deforms, such that the first elastic resin material is molten,
the applicator applies the first elastic resin material, which has been discharged from the first discharge port, in a net form to the cooling member,
the cooling member cools the first elastic resin material, which has been applied to the cooling member, to the first temperature range such that the first elastic resin material is solidified so as to form a net-shaped elastic sheet, and
the laminator laminates and bonds the elastic sheet to a nonwoven fabric.

19. The stretchable composite sheet manufacturing apparatus according to claim 18, wherein
the applicator includes a second discharger,
the cooling member is disposed below the second discharger,
the second discharger includes a plurality of second discharge ports to discharge second elastic resin materials in a linear form, the second elastic resin materials each being composed mainly of a second thermoplastic elastic resin, the second elastic resin materials being heated to a temperature higher than a second temperature range in which the second elastic resin materials elastically deform, such that the second elastic resin materials are molten,
the applicator applies the second elastic resin materials, which have been discharged from the second discharge ports, in a linear form to the cooling member such that the second elastic resin materials are in parallel with each other and overlap with the first elastic resin material applied to the cooling member, and
the cooling member cools the second elastic resin materials, which have been applied to the cooling member, to the second temperature range such that the second elastic resin materials are solidified.

20. The stretchable composite sheet manufacturing apparatus according to claim 18, further comprising an additional cooling member along which the elastic sheet delivered out of the cooling member passes, wherein
the applicator includes a second discharger,
the additional cooling member is disposed below the second discharger,
the second discharger includes a second discharge port to discharge a second elastic resin material in a linear or fibrous form, the second elastic resin material being composed mainly of a second thermoplastic elastic resin, the second elastic resin material being heated to a temperature higher than a second temperature range in which the second elastic resin material elastically deforms, such that the second elastic resin material is molten,
the applicator applies the second elastic resin material, which has been discharged from the second discharge port, in a linear or net form to the additional cooling member, and
the additional cooling member cools the second elastic resin material, which has been applied to the additional cooling member, to the second temperature range such that the second elastic resin material is solidified.

21. The stretchable composite sheet manufacturing apparatus according to any one of claims 18 to 20, further comprising a stretcher to stretch the elastic sheet, wherein
the laminator bonds the stretched elastic sheet to the nonwoven fabric.

22. A stretchable composite sheet comprising:
a first nonwoven fabric; and
a net-shaped elastic sheet which is composed mainly of a thermoplastic elastic resin and whose fibrous or linear elements partially overlap with each other, wherein
the elastic sheet is bonded to the first nonwoven fabric at a plurality of locations away from each other.

23. The stretchable composite sheet according to claim 22, further comprising a second nonwoven fabric, wherein
the second nonwoven fabric is laminated to the elastic sheet and the first nonwoven fabric such that the elastic sheet is sandwiched between the first nonwoven fabric and the second nonwoven fabric, and
the second nonwoven fabric is bonded to at least either one of the elastic sheet and the first nonwoven fabric at a plurality of locations away from each other.
